# EUROPÄISCHE PATENTSCHRIFT

(11) **EP 1 387 828 B1**
(45) Veröffentlichungstag und Bekanntmachung des Hinweises auf die Patenterteilung: **12.10.2005**
(21) Anmeldenummer: 02735369.7
(22) Anmeldetag: 17.05.2002
(51) Int. Cl.: C07D 211/00

(54) **VERFAHREN ZUR HERSTELLUNG VON BIPERIDEN IV**
METHOD FOR PRODUCING BIPERIDEN IV
PROCEDE POUR PRODUIRE DU BIPERIDENE IV

(30) Priorität: 18.05.2001 DE 10124453
(43) Veröffentlichungstag der Anmeldung: 11.02.2004
(73) Patentinhaber: LABORATORIO FARMACEUTICO S.I.T. SPECIALITA' IGIENICO TERAPEUTICHE S.r.l., 27035 Mede (Pavia) (IT)
(72) Erfinder: GROSSE, Markus, 68723 Schwetzingen (DE); WEBER, Klaus, Dieter, 67346 Speyer (DE); THYES, Marco, 67061 Ludwigshafen (DE); KLEIN, Peter, 67134 Birkenheide (DE); VILSMAIER, Elmar, 67731 Otterbach (DE)
(74) Vertreter: De Gregori, Antonella
(86) Internationale Anmeldenummer: PCT/EP2002/005497
(87) Internationale Veröffentlichungsnummer: WO 2002/096874

(56) Entgegenhaltungen:
- DE-B- 1 005 067
- US-A- 2 789 110
- COREY, E. J. ET AL: "Enantioselective total synthesis of (-)-preclavulone A" TETRAHEDRON LETT. (1988), 29(9), 995-8 , XP002210239
- ELTZE, M; FIGULA, V.: "Affinity and selectivity of biperiden enantiomers for muscarinic receptor subtypes" EUROPEAN JOURNAL OF PHARMACOLOGY, Bd. 158, 1988, Seiten 11-19, XP002206676

## Beschreibung

Die vorliegende Erfindung betrifft ein Verfahren zur Herstellung von Biperiden.

Biperiden ist ein bekanntes zentrales Anticholinergikum und wird zur Behandlung der Parkinsonschen Krankheit eingesetzt (Ullmanns Enzyklopädie der technischen Chemie, 4. Aufl., Band 21, Verlag Chemie, 1982, S. 627). Es handelt sich um ein Racemat aus 1-(Bicyclo[2.2.1]hept-5-en-2-yl(exo,R))-1-phenyl-3-piperidinopropanol(1,S) und 1-(Bicyclo[2.2.1]hept-5-en-2-yl(exo,S))-1-phenyl-3-piperidinopropanol(1,R) (Ia) und stellt eines von vier möglichen Enantiomerenpaaren (Ia-d) des Aminoalkohols 1-(Bicyclo[2.2.1]hept-5-en-2-yl)-1-phenyl-3-piperidino-1-propanol (I) dar.

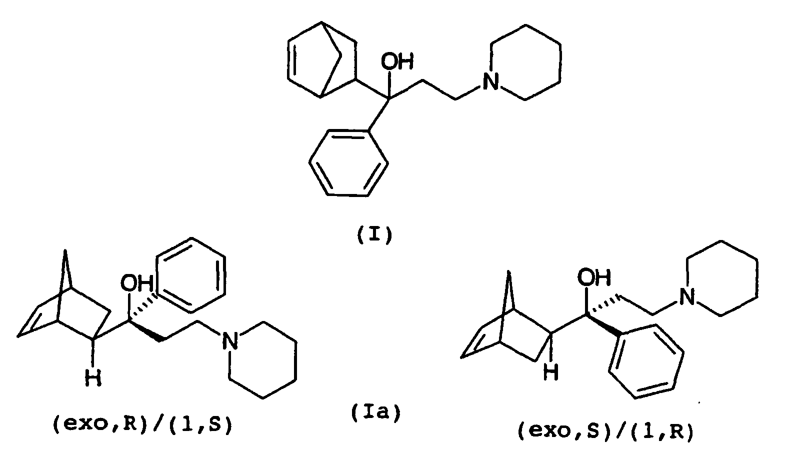

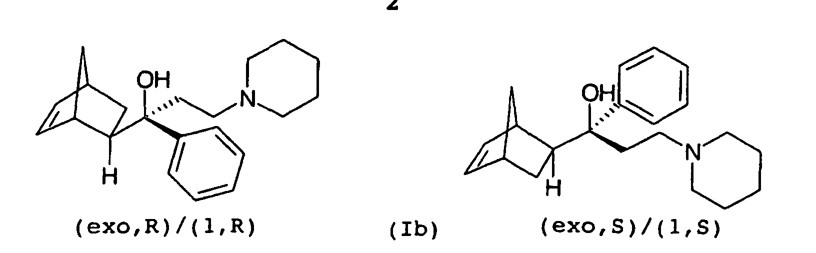

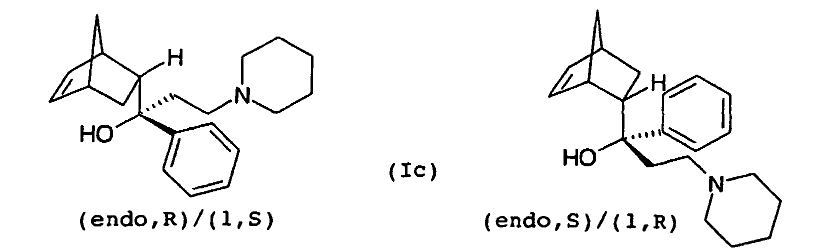

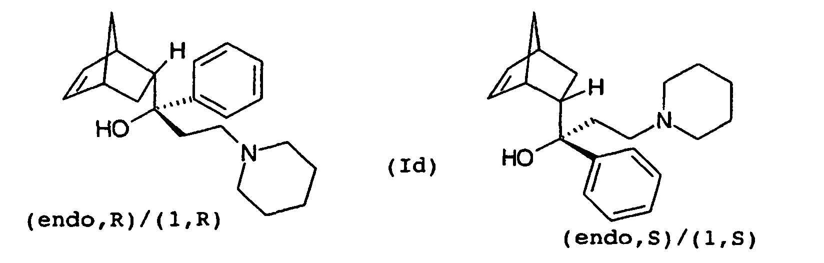

Die DE 1 005 067 und die US 2,789,110 beschreiben die Herstellung des Aminoalkohols I durch Umsetzung von 1-(Bicyclo[2.2.1]hept-5-en-2-yl)-3-piperidino-1-propanon (II) mit einem Phenylmagnesiumhalogenid. Die US 2,789,110 beschreibt zudem die Herstellung des Propanons II, ausgehend von 1-(Bicyclo[2.2.1]hept-5-en-2-yl)-ethanon (III), Paraformaldehyd und Piperidinhydrochlorid in einer Reaktion nach Mannich sowie die Herstellung des Ethanons III aus Cyclopentadien und Methylvinylketon in einer Cycloaddition nach Diels und Alder.

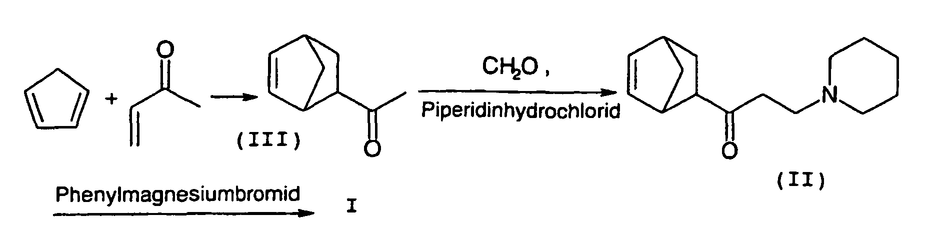

Weder die DE 1 005 067 noch die US 2,789,110 offenbaren, ob es sich bei dem so erhaltenen Aminoalkohol I um ein Isomerengemisch oder um ein reines Isomer handelt.

Das Edukt der Propanolherstellung, 1-(Bicyclo[2.2.1]hept-5-en-2-yl)-3-piperidino-1-propanon (II), kann in zwei isomeren Formen, als exo- oder als endo-Isomeres (II-exo, II-endo), vorliegen, wobei nur die exo-Form in der oben genannten Reaktion mit einem Phenylmagnesiumhalogenid Biperiden ergeben kann.

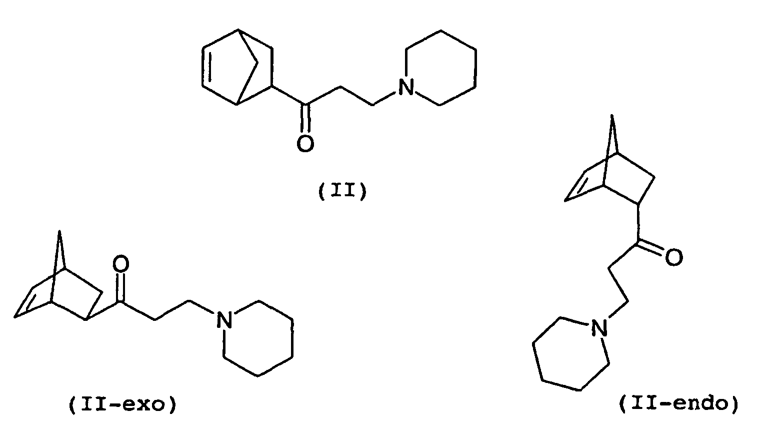

Die Strukturformeln des II-exo und des II-endo zeigen der Einfachheit halber jeweils nur eines von zwei möglichen Enantiomeren des exo- bzw. endo-Isomers. Im Folgenden bezieht sich die Bezeichnung II-exo bzw. II-endo jedoch jeweils auf das Enantiomerenpaar der exo- bzw. endo-Form.

Auch 1-(Bicyclo[2.2.1]hept-5-en-2-yl)ethanon (III), die Ausgangssubstanz für die Synthese des Propanons II, kann sowohl als exoals auch als endo-Isomeres vorliegen (III-exo, III-endo) und entsprechend führt nur die Umsetzung des exo-Isomeren in den Folgeschritten zu Biperiden.

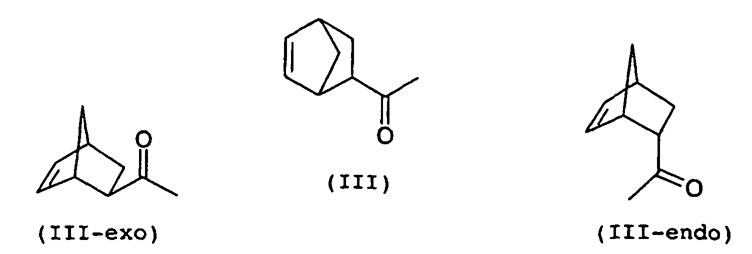

Die Strukturformeln des III-exo und des III-endo zeigen der Einfachheit halber jeweils nur eines von zwei möglichen Enantiomeren des exo- bzw. endo-Isomers. Im Folgenden bezieht sich die Bezeichnung III-exo bzw. III-endo jedoch auf das Enantiomerenpaar der exo- bzw. endo-Form.

Keiner der oben genannten Schriften lassen sich Angaben zur Konfiguration der eingesetzten Edukte III und Zwischenprodukte II entnehmen.

Es ist bekannt, dass das 1-(Bicyclo[2.2.1]hept-5-en-2-yl)ethanon (III) aus der Cycloaddition in einem exo/endo-Verhältnis von 1:4 erhalten wird (z.B. R. Breslow, U. Maitra, Tetrahedron Letters, 1984, 25, 1239). Da der eingangs genannte Stand der Technik keinerlei Angaben zur Stereochemie des Ethanons III macht, ist davon auszugehen, dass das Ethanon III in diesem Isomerenverhältnis zur Herstellung des Aminoalkohols I eingesetzt wurde.

Die Herstellung von exo-1-(Bicyclo[2.2.1]hept-5-en-2-yl)ethanon (III-exo) ist 1965 von J.G. Dinwiddie und S.P. McManus beschrieben worden (J. Org. Chem., 1965, 30, 766). Dabei werden exo/endo-Gemische von 1-(Bicyclo[2.2.1]hept-5-en-2-yl)ethanon (III), in denen der endo-Anteil überwiegt, in Methanol in Gegenwart von Natriummethanolat erhitzt. Sie isomerisieren hierbei zu Gemischen mit einem exo-Anteil von ca. 70 %. Aus diesen kann durch fraktionierte Destillation und gegebenenfalls Redestillation des Destillats exo-1-(Bicyclo[2.2.1]hept-5-en-2-yl)ethanon (III-exo) mit einem Reinheitsgrad von bis zu 95 % erhalten werden.

Versuche der Anmelderin haben gezeigt, dass auch bei der Verwendung von einheitlichem exo-Ethanon III-exo als Ausgangsmaterial in der Reaktion nach Mannich stets ein exo/endo-Gemisch des Propanons II entsteht. Dies ist bei der sich anschließenden Umsetzung des Propanons II zum Propanol I hinsichtlich der Ausbeute an sauberem Biperiden (Ia) von Nachteil. Unter sauberem Biperiden versteht man ein Biperiden (Ia) mit einer Reinheit von wenigstens 99,0 %, wie sie für pharmazeutische Anwendungen in der Regel erforderlich ist.

Der vorliegenden Erfindung liegt die Aufgabe zugrunde, ein Verfahren zur Herstellung von Biperiden bereit zu stellen, das dieses in einer höheren Ausbeute liefert, wobei dieses Verfahren ein Verfahren zur Herstellung von möglichst isomerenreinem exo-Propanon II-exo umfasst. Unter dem exo-Propanon II-exo soll ein Propanon II verstanden werden, das zu wenigstens 96 %, vorzugsweise zu wenigstens 97 % und besonders bevorzugt zu wenigstens 98 %, aus dem exo-Isomeren II-exo besteht. Unter Biperiden soll eine Substanz der Strukturformel Ia verstanden werden.

Die Aufgabe konnte gelöst werden durch ein Verfahren zur Herstellung von Biperiden (Ia) durch Umsetzung von exo-1-(Bicyclo-[2.2.1]hept-5-en-2-yl)-3-piperidino-1-propanon (II-exo) mit einer Phenylmagnesiumverbindung, dadurch gekennzeichnet, dass die Herstellung des exo-1-(Bicyclo[2.2.1]hept-5-en-2-yl)-3-piperidino-1-propanons (II-exo) die folgenden Schritte umfasst:
a) Überführung von exo-1-(Bicyclo[2.2.1]hept-5-en-2-yl)ethanon (III-exo) in einen exo-Silylenolether IV, 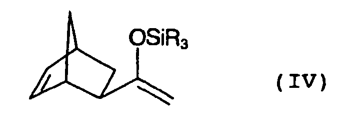 worin R gleich oder verschieden sein kann und für eine Alkylgruppe oder eine Cycloalkylgruppe steht, und
b) Umsetzung des exo-Silylenolethers IV mit einer N-Methylenpiperidinium-Verbindung.

Die Strukturformel des exo-Silylenolethers IV zeigt der Einfachheit halber nur eines von zwei möglichen Enantiomeren. Im Folgenden bezieht sich die Bezeichnung exo-Silylenolether IV jedoch auf das Enantiomerenpaar.

Unter exo-1-(Bicyclo[2.2.1]hept-5-en-2-yl)ethanon (III-exo) soll im Folgenden ein Ethanon III verstanden werden, das zu wenigstens 96 %, vorzugsweise zu wenigstens 97 % und besonders bevorzugt zu wenigstens 98 %, aus dem exo-Isomeren III-exo besteht. Entsprechendes gilt für den exo-Silylenolether IV.

Bei den im erfindungsgemäßen Verfahren eingesetzten exo-Isomeren handelt es sich, wie bereits für das exo-Ethanon III-exo, das exo-Propanon II-exo bzw. für den exo-Silylenolether IV beschrieben, um Enantiomerenpaare. Um zu Biperiden (Ia) zu gelangen, das selbst ein Racemat ist, werden racemische Enantiomerengemische der Ausgangsstoffe und der Zwischenprodukte eingesetzt. Das erfindungsgemäße Verfahren kann jedoch auch auf reine Enantiomere sowie auf nicht racemische Enantiomerengemische angewendet werden.

Zur Überführung des exo-Ethanons III-exo in den entsprechenden exo-Silylenolether IV wird das exo-Ethanon III-exo im Allgemeinen zuerst mit einer Base in das exo-Enolat V überführt und dieses anschließend mit einer geeigneten Silylverbindung umgesetzt.

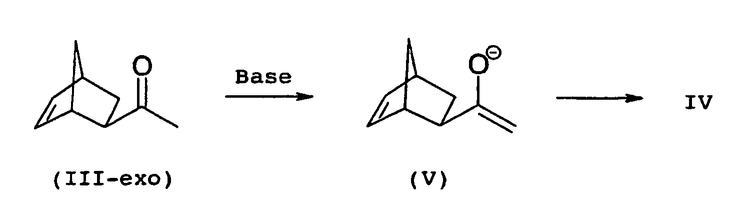

Die Strukturformel des exo-Enolats V zeigt der Einfachheit halber nur eines von zwei möglichen Enantiomeren. Im Folgenden bezieht sich die Bezeichnung exo-Enolat V jedoch auf das Enantiomerenpaar.

Unter exo-Enolat V soll im Folgenden ein Enolat V verstanden werden, das zu wenigstens 96 %, vorzugsweise zu wenigstens 97 % und besonders bevorzugt zu wenigstens 98 %, aus dem exo-Isomeren besteht.

Geeignete Silylverbindungen für die Umsetzung mit dem exo-Enolat V sind die Verbindungen der allgemeinen Formel R₃Si-X, worin R die zuvor genannten Bedeutungen hat und X für eine nucleophil verdrängbare Abgangsgruppe, vorzugsweise für ein Halogenatom und insbesondere für Chlor, steht. Alkyl weist in diesem Zusammenhang vorzugsweise 1 bis 4 C-Atome auf, d. h. ist unter Methyl, Ethyl, n-Propyl, Isopropyl, n-Butyl, Isobutyl, sek-Butyl oder tert-Butyl ausgewählt. Cycloalkyl weist in diesem Zusammenhang vorzugsweise 5 bis 8 C-Atome auf wie Cyclopentyl, Cyclohexyl, Cycloheptyl oder Cyclooctyl. Besonders bevorzugte Silylverbindungen sind die Tri-C₁-C₄-alkylsilylhalogenide, insbesondere werden Tri-C₁-C₄-alkylsilylchloride verwendet. Besonders bevorzugt wird Trimethylsilylchlorid eingesetzt.

Das exo-Ethanon III-exo und die Silylverbindung werden in der Regel in einem Molverhältnis im Bereich von 1:1 bis 1:2 eingesetzt. Vorzugsweise wird die Silylverbindung im Überschuss eingesetzt, bevorzugt in einem Überschuss von 10 bis 100 Mol-%, insbesondere von 10 bis 30 Mol-%, z. B. von 20 Mol-%.

Die Umsetzung erfolgt im Allgemeinen bei einer Temperatur im Bereich von -100 bis 0 °C, vorzugsweise von -85 bis -10 °C und besonders bevorzugt von -80 bis -60 °C, z. B. bei -78 °C.

Bei der Behandlung des exo-Ethanons III-exo mit einer Base zur Überführung in das exo-Enolat V verwendet man als Basen in der Regel Metallamide. Bevorzugt verwendet man Alkalimetallamide und insbesondere Lithiumamide. Das Amid-Stickstoffatom ist vorzugsweise ein- oder zweifach substituiert. Geeignete Substituenten am Amidstickstoff sind C₁-C₄-Alkylreste wie Methyl, Ethyl, n-Propyl, Isopropyl, n-Butyl, sek-Butyl oder tert-Butyl, weiterhin C₅-C₈-Cycloalkylreste wie Cyclopentyl, Cyclohexyl, Cycloheptyl oder Cyclooctyl, die ihrerseits 1 bis 4 Methylgruppen aufweisen können, und Tri-C₁-C₄-alkylsilylreste wie Trimethylsilyl oder Triisopropylsilyl. Außerdem kann der Amidstickstoff dergestalt zweifach substituiert sein, dass er Bestandteil eines gesättigten, 5- oder 6-gliedrigen Heterocyclus ist, der seinerseits durch 1, 2, 3 oder 4 C₁-C₄-Alkylgruppen, insbesondere Methylgruppen, substituiert sein kann wie beispielsweise in den Amiden des Piperidins, 2,2,6,6-Tetramethylpiperidins oder des Pyrrolidins. Vorzugsweise ist der Amidstickstoff zweifach substituiert. Besonders bevorzugt setzt man Lithiumdiisopropylamid als Base ein.

Die Umsetzung des exo-Ethanons III-exo mit der Base erfolgt im Allgemeinen in einem geeigneten Lösungsmittel in einem Molverhältnis des exo-Ethanons III-exo zur Base im Bereich von 1:1 bis 1:1,5, vorzugsweise von 1:1 bis 1:1,2 und besonders bevorzugt nahezu äquimolar.

In der Regel führt man die Umsetzung des exo-Ethanons III-exo mit der Base in einem inerten Lösungsmittel durch. Hierfür geeignete Lösungsmittel sind C₅-C₉-Aliphaten wie n-Hexan oder n-Heptan, Aromaten wie Benzol, Toluol, Xylole oder Ethylbenzol, aliphatische C₄-C₈-Ether wie Diethylether, Diisopropylether, tert-Butylmethylether oder 1,2-Dimethoxyethan, alicyclische C₄-C₆-Ether wie Tetrahydrofuran oder Dioxan oder Gemische davon. Die Lösungsmittel werden in der Regel, wie für solche Umsetzungen üblich, wasserfrei eingesetzt. In einer speziellen Ausgestaltungsform des erfindungsgemäßen Verfahrens setzt man bei der Verwendung von Lithiumdiisopropylamid als Base ein Gemisch aus Tetrahydrofuran, Ethylbenzol und n-Heptan als Lösungsmittel ein.

Die Umsetzung des exo-Ethanons III-exo mit der Base erfolgt in der Regel bei einer Temperatur im Bereich von -100 bis 0 °C, vorzugsweise von -85 bis -10 °C und besonders bevorzugt von -80 bis -60 °C, z. B. bei -78 °C. Bei der Umsetzung kann man das exo-Ethanon III-exo vorlegen und die Base hinzufügen oder umgekehrt die Base vorlegen und das exo-Ethanon III-exo hinzufügen, wobei die letzte Möglichkeit bevorzugt ist. Hierzu legt man geeigneterweise eine Lösung der Base in einem oder mehreren der oben genannten Lösungsmittel vor und fügt das exo-Ethanon III-exo bei den oben beschriebenen Temperaturen hinzu. Die Konzentration der Base in der Vorlage beträgt üblicherweise 0,1 bis 10 mol/l, vorzugsweise 1 bis 3 mol/l. Die Zugabe des exo-Ethanons III-exo erfolgt im Allgemeinen portionsweise; das exo-Ethanon III-exo wird dabei unverdünnt oder in einem oder mehreren der oben genannten Lösungsmittel, vorzugsweise in demselben/denselben Lösungsmittel(n), in dem/denen die Base vorgelegt wird, in einer Konzentration von 0,1 bis 20 mol/l, vorzugsweise von 1 bis 15 mol/l, gelöst hinzugefügt. Vorzugsweise wird das exo-Ethanon III-exo jedoch in Reinform zugegeben. Zur Vervollständigung der Reaktion kann das Gemisch im oben definierten Temperaturbereich 10 Minuten bis z. B. 5 Stunden, vorzugsweise 30 Minuten bis eine Stunde belassen werden, wobei vorzugsweise gerührt wird.

Zu dem so erhaltenen Reaktionsgemisch, das das exo-Enolat V enthält, fügt man üblicherweise in situ, d. h. ohne vorheriges Isolieren des Enolats, eine der oben beschriebenen siliziumorganischen Verbindungen im oben definierten Temperaturbereich hinzu. Die Silylverbindung kann auf einmal oder vorzugsweise über einen Zeitraum von 5 Minuten bis zu mehreren Stunden, insbesondere von 10 Minuten bis zu einer Stunde, unverdünnt oder in einem oder mehreren der oben genannten Lösungsmittel gelöst, zugegeben werden. Vorzugsweise wird die Silylverbindung in Reinform zugegeben. Bei der Zugabe als Lösung beträgt die Konzentration der Silylverbindung in der Regel 0,1 bis 20 mol/l, vorzugsweise 1 bis 15 mol/l. Zur Vervollständigung der Reaktion wird in der Regel das Reaktionsgemisch noch einige Zeit belassen, z. B. 1 bis 5 Stunden, wobei vorzugsweise gerührt wird. Dabei kann man die Temperatur des Gemischs bei den oben genannten Werten belassen oder vorzugsweise Raumtemperatur erreichen lassen, z. B. durch Entfernen der Kühlvorrichtung.

Sowohl die Herstellung des exo-Enolats V als auch dessen Umsetzung zum exo-Silylenolether IV erfolgen geeigneterweise unter Inertgasatmosphäre. Als Inertgase kommen beispielsweise Stickstoff und die Edelgase wie Argon in Betracht.

Die Aufarbeitung des Reaktionsgemischs erfolgt vorzugsweise wässrig-extraktiv. Der daraus erhaltene rohe exo-Silylenolether IV wird gegebenenfalls destillativ, vorzugsweise im Vakuum, gereinigt. Der hierbei erhaltene exo-Silylenolether IV ist neu und als wertvolles Zwischenprodukt zur Herstellung von Biperiden (Ia) ebenfalls Gegenstand der vorliegenden Erfindung.

Entsprechende Silylether in der endo-Form sind aus der Literatur bekannt (siehe Corey, E. J., Xiang, Yi Bin, Tetrahedron Letters 29, 1988, 995-998).

Die sich anschließende Umsetzung des exo-Silylenolethers IV mit einer N-Methylenpiperidinium-Verbindung zum exo-Propanon II-exo erfolgt im Allgemeinen in einem geeigneten polaren aprotischen organischen Lösungsmittel. Geeignete polare aprotische organische Lösungsmittel sind unter anderem aliphatische C₄-C₈-Ether wie Diethylether, Diisopropylether oder 1,2-Dimethoxyethan, alicyclische C₄-C₆-Ether wie Tetrahydrofuran oder Dioxan, chlorierte C₁-C₂-Aliphaten wie Dichlormethan, Carbonsäurederivate wie Acetonitril, N,N-Dimethylformamid oder N-Alkylpyrrolidone, z. B. N-Methyl-2-pyrrolidon, und Sulfoxide wie Dimethylsulfoxid. Vorzugsweise verwendet man N,N-Dimethylformamid oder N-Methyl-2-pyrrolidon. Die Lösungsmittel werden in der Regel, wie für Umsetzungen mit N-Methylenpiperidiniumverbindungen üblich, wasserfrei eingesetzt.

Der exo-Silylenolether IV und die N-Methylenpiperidinium-Verbindung werden dabei vorzugsweise in einem Molverhältnis von IV zu N-Methylenpiperidinium-Verbindung im Bereich von 1:1 bis 1:2 eingesetzt. Die N-Methylenpiperidinium-Verbindung wird insbesondere im Überschuss, z. B. in einem Überschuss von 10 bis 100 Mol-%, besonders bevorzugt 20 bis 70 Mol-%, z. B. 50 Mol-%, bezogen auf IV, eingesetzt.

Vorzugsweise legt man den exo-Silylenolether IV im Lösungsmittel bei einer Temperatur im Bereich von -60 bis 10 °C, insbesondere von -40 bis 0 °C und besonders bevorzugt von -30 bis -15 °C vor und gibt dann bei diesen Temperaturen die N-Methylenpiperidinium-Verbindung zu. Die Zugabe kann in einer Portion oder über einen Zeitraum von 5 Minuten bis zu mehreren Stunden erfolgen. Zur Vervollständigung der Reaktion wird das Gemisch geeigneterweise noch einige Zeit, z. B. 15 Minuten bis 5 Stunden, belassen, wobei vorzugsweise gerührt wird. Dabei kann man das Gemisch im oben definierten Temperaturbereich belassen oder es vorzugsweise Raumtemperatur erreichen lassen.

Selbstverständlich kann man auch die N-Methylenpiperidinium-Verbindüng vorlegen und den exo-Silylenolether IV hinzufügen, wobei die Zugabe der N-Methylenpiperidinium-Verbindung zum exo-Silylenolether IV bevorzugt ist.

Die Isolierung des exo-Propanons II-exo aus dem Reaktionsgemisch erfolgt in der Regel wässrig-extraktiv. Hierzu wird das mit Wasser versetzte Reaktionsgemisch zuerst bei einem pH-Wert von 2 bis 6, vorzugsweise von 2 bis 4, z. B. 3, zwecks Reinigung mit einem mit Wasser begrenzt oder nicht mischbaren Lösungsmittel gewaschen. Zu den geeigneten, mit Wasser begrenzt oder nicht mischbaren organischen Lösungsmitteln zählen C₅-C₈-Aliphaten wie n-Pentan oder n-Hexan, C₅-C₆-Alicyclen wie Cyclohexan, Aromaten wie Benzol, Toluol oder Xylole, aliphatische C₄-C₈-Ether wie Diethylether, tert-Butylmethylether oder Diisopropylether bzw. Gemische davon. Vorzugsweise werden aliphatische C₄-C₈-Ether wie Diisopropylether verwendet.

Nach dem Waschen wird die wässrige Phase auf einen pH-Wert von in der Regel 7,5 bis 12, vorzugsweise von 9 bis 11, z. B. 10, mit einer geeigneten Base, vorzugsweise in Form ihrer wässrigen Lösung, eingestellt und mit einem der oben genannten Lösungsmittel gegebenenfalls mehrfach extrahiert. Als Basen eignen sich unter anderem Alkali- oder Erdalkalihydroxide oder Alkalicarbonate. Bevorzugt verwendet man Alkalihydroxide bzw. ihre wässrigen Lösungen, insbesondere Kaliumhydroxid bzw. Kalilauge oder Natriumhydroxid bzw. Natronlauge. Nach dem Entfernen des Lösungsmittels aus dem/den alkalischen Extrakt(en), was z. B. destillativ, vorzugsweise unter vermindertem Druck, erfolgt, erhält man ein Propanon II, das zu wenigstens 96 % aus dem exo-Isomeren II-exo besteht. Das Propanon II weist somit ein exo/endo-Verhältnis von wenigstens 24:1 auf.

Der Anteil des exo-Isomeren II-exo am Propanon II hängt dabei nahezu ausschließlich vom Reinheitsgrad des eingesetzten exo-Ethanons III-exo ab. Bei Einsatz eines Ethanons III mit einem exo-Anteil von 100 % resultiert somit ein Propanon II mit einem exo-Anteil von etwa 100 %.

Bei der erfindungsgemäßen Umsetzung des exo-Ethanons III-exo zum Propanon II erhält man dieses nicht nur in Form des exo-Isomeren II-exo, ohne dass Isomerisierung beobachtet wird, sondern auch in einer wesentlich größeren Ausbeute im Vergleich zur üblichen Vorgehensweise.

Hier und im Folgenden steht die Bezeichnung exo-1-(Bicyclo-[2.2.1]hept-5-en-2-yl)-3-piperidino-1-propanon (II-exo) für ein Propanon II, das zu 96 bis 100 % aus dem exo-Propanon II-exo besteht.

Das erfindungsgemäß hergestellte exo-1-(Bicyclo[2.2.1]hept-5-en-2-yl)-3-piperidino-1-propanon (II-exo) wird anschließend zur Herstellung des Biperidens mit einer Phenylmagnesiumverbindung im Sinne einer Grignard-Reaktion umgesetzt. Bevorzugte Phenylmagnesiumverbindungen sind Diphenylmagnesium und besonders bevorzugt Phenylmagnesiumverbindung der allgemeinen Formel 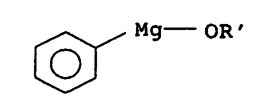 worin R' für C₁-C₄-Alkyl, wie Methyl, Ethyl, n-Propyl, Isopropyl oder n-Butyl, C₄-C₆-Cycloalkyl, wie Cyclohexyl, C₄-C₆-Cycloalkyl-C₁-C₄-alkyl, wie 2-Cyclohexylethyl, Phenyl-C₁-C₄-alkyl, wie Benzyl, 2-Phenylethyl oder 3-Phenylpropyl, substituiertes Phenyl-C₁-C₄-alkyl, wie 3,4-(Methylendioxy)benzyl, Heteroaryl, wie 8-Chinolyl, Heteroaryl-C₁-C₄-alkyl, wie Furfuryl, 2-Thienylmethyl oder 2-(2-Thienyl)ethyl, oder Benzhydryl steht. Die Umsetzung wird man üblicherweise in einem für Grignard-Reaktionen geeigneten Lösungsmittel durchführen. Die Phenylmagnesiumverbindung der oben dargestellten Formel wird im Folgenden als Phenylmagnesiumalkoxid bezeichnet.

Geeignete Lösungsmittel sind Aromaten wie Benzol, Toluol oder Xylole, acyclische oder cyclische Ether mit 4 bis 6 C-Atomen, Gemische davon oder deren Mischungen mit aliphatischen oder alicyclischen Kohlenwasserstoffen wie n-Hexan bzw. Cyclohexan. Geeignete acyclische Ether sind z. B. Diethylether und tert-Butylmethylether, geeignete cyclische Ether sind z. B. Tetrahydrofuran und Dioxan. Bevorzugt verwendet man Diethylether, Tetrahydrofuran oder Dioxan oder Mischungen davon. Die Lösungsmittel werden in der Regel, wie für Grignard-Reaktionen üblich, wasserfrei eingesetzt.

Das Phenylmagnesiumalkoxid wird in allgemein bekannter Weise, z. B. durch Umsetzung von Diphenylmagnesium mit einem Alkohol der allgemeinen Formel R'OH, worin R' wie oben definiert ist, hergestellt. Diphenylmagnesium und der Alkohol werden dabei in einem Molverhältnis im Bereich von 1:0,9 bis 1:1,5, vorzugsweise im Bereich von 1:1 bis 1:1,2 und besonders bevorzugt etwa äquimolar umgesetzt. Gewöhnlich wird Diphenylmagnesium, das üblicherweise wie weiter unten beschrieben in situ erzeugt wird, in einem der oben genannten, für Grignard-Reaktionen geeigneten Lösungsmittel vorgelegt und der Alkohol in der Regel portionsweise über eine Zeitspanne von 5. Minuten bis etwa eine Stunde bei einer Temperatur von 0 bis 80 °C, vorzugsweise von 0 bis 50 °C und besonders bevorzugt von 0 bis 40 °C zugegeben. Nach beendeter Zugabe kann man das Gemisch im selben Temperaturbereich zur Vervollständigung der Reaktion noch 15 Minuten bis 2 Stunden, vorzugsweise 15 Minuten bis eine Stunde, belassen oder vorzugsweise rühren.

Die Herstellung des in dem erfindungsgemäßen Verfahren eingesetzten Diphenylmagnesiums erfolgt in an sich bekannter Weise. Beispielsweise kann man ein Phenylmagnesiumhalogenid, z. B. Phenylmagnesiumchlorid, in einem geeigneten Lösungsmittel mit Dioxan versetzen, wobei unter Verschiebung des Schlenk-Gleichgewichts Diphenylmagnesium und der entsprechende Magnesiumhalogenid-Dioxan-Komplex entstehen. Letzterer fällt in der Regel aus, wird aber bevorzugt nicht aus der Lösung entfernt. Geeignete Lösungsmittel sind im Allgemeinen acyclische und cyclische Ether mit vorzugsweise 4 bis 6 C-Atomen oder deren Mischungen mit aliphatischen, alicyclischen oder aromatischen Kohlenwasserstoffen. Geeignete acyclische Ether sind z. B. Diethylether und tert-Butylmethylether, ein geeigneter cyclischer Ether ist Tetrahydrofuran. Zu den geeigneten aliphatischen bzw. alicyclischen Kohlenwasserstoffen zählen insbesondere n-Hexan bzw. Cyclohexan, geeignete aromatische Kohlenwasserstoffe sind z. B. Benzol, Toluol und Xylole.

Dioxan wird in der Regel mindestens äquimolar im Verhältnis zum Phenylmagnesiumhalogenid eingesetzt. Soll als Phenylmagnesiumverbindung Diphenylmagnesium verwendet werden, so setzt man vorzugsweise Dioxan im Überschuss, beispielsweise in einem Überschuss von 50 bis 500 Mol-%, insbesondere von 100 bis 300 Mol-% und speziell von 100 bis 200 Mol-%, ein. Soll Diphenylmagnesium zuerst in das Phenylmagnesiumalkoxid überführt werden, setzt man vorzugsweise Dioxan und das Phenylmagnesiumhalogenid in einem Molverhältnis im Bereich von 1:1 bis 1,5:1, insbesondere 1:1 bis 1,2:1 und besonders bevorzugt etwa äquimolar ein.

Die Zugabe des Dioxans zu der Lösung des Phenylmagnesiumhalogenids erfolgt in der Regel bei einer Temperatur im Bereich von -20 bis 60 °C, vorzugsweise im Bereich von -10 bis 40 °C.

Üblicherweise belässt man die nach Zugabe des Dioxans erhaltene Mischung noch 15 Minuten bis 2 Stunden, vorzugsweise 20 Minuten bis eine Stunde, in dem für die Zugabe des Dioxans genannten Temperaturbereich, bevor man sie ins erfindungsgemäße Verfahren einsetzt.

Sowohl die Herstellung von Diphenylmagnesium, die Umsetzung zum Phenylmagnesiumalkoxid als auch die Umsetzung mit dem exo-Propanon II-exo nach Grignard erfolgen geeigneterweise unter einer Inertgasatmosphäre. Als Inertgase kommen beispielsweise Stickstoff und die Edelgase wie Argon sowie deren Mischungen in Betracht.

In der Grignard-Reaktion des exo-Propanons II-exo mit der Phenylmagnesiumverbindung setzt man in der Regel die Phenylmagnesiumverbindung und das exo-Propanon II-exo in einem Molverhältnis im Bereich von 0,8:1 bis 3:1, vorzugsweise im Bereich von 0,8:1 bis 2:1 und insbesondere im Bereich von 0,8:1 bis 1,5:1 ein. Besonders bevorzugt wird das exo-Propanon II-exo mit Diphenylmagnesium bzw. mit dem Phenylmagnesiumalkoxid in einem Molverhältnis im Bereich von 1:1 bis 1:1,3 umgesetzt.

In der Regel legt man die Phenylmagnesiumverbindung in Form einer Lösung in einem der oben genannten, für Grignard-Reaktionen geeigneten, organischen Lösungsmittel vor und fügt das exo-Propanon II-exo bei einer Temperatur im Bereich von -20 °C bis zur Siedetemperatur, vorzugsweise im Bereich von -10 °C bis 90 °C und besonders bevorzugt im Bereich von 0 °C bis 70 °C hinzu. Dabei wird die Phenylmagnesiumverbindung in der Regel in einer Konzentration im Bereich von 0,1 bis 10 mol/l, vorzugsweise im Bereich von 0,1 bis 3 mol/l und besonders bevorzugt im Bereich von 0,2 bis 2 mol/l eingesetzt.

Die Zugabe des exo-Propanons II-exo kann in einer Portion oder vorzugsweise über einen Zeitraum von wenigen Minuten bis zu mehreren Stunden, z. B. 5 Minuten bis 5 Stunden, erfolgen. Die Zugabe des exo-Propanons II-exo erfolgt entweder in Form einer Lösung in einem der oben genannten, für Grignard-Reaktionen geeigneten, inerten Lösungsmittel oder vorzugsweise in Reinform. Bei der Zugabe als Lösung beträgt die Konzentration des exo-Propanons II-exo in der Regel 0,1 bis 20 mol/l, vorzugsweise 1 bis 15 mol/l. Zur Vervollständigung der Reaktion wird das Reaktionsgemisch üblicherweise 15 Minuten bis 5 Stunden, speziell 30 Minuten bis 2 Stunden, bei einer Temperatur im Bereich von -20 °C bis zur Siedetemperatur des Reaktionsgemischs, vorzugsweise im Bereich von -10 °C bis 90 °C und besonders bevorzugt im Bereich von 10 °C bis 80 °C belassen, wobei man vorzugsweise zur besseren Durchmischung rührt. Die Aufarbeitung erfolgt wie für Grignard-Reaktionen üblich wässrig-extraktiv, z. B. indem man das Reaktionsgemisch mit Wasser, einer wässrigen Ammoniumchloridlösung oder einer sauren wässrigen Lösung quencht, wobei man im letzten Fall in der Regel den pH-Wert des entstandenen Gemischs anschließend alkalisch einstellt, das gequenchte Gemisch gegebenenfalls nach Abtrennung einer organischen Phase mit einem mit Wasser nicht mischbaren, zum Lösen des Produkts geeigneten Lösungsmittel extrahiert und aus dem Extrakt bzw. aus dem mit der organischen Phase vereinigten Extrakt das Lösungsmittel entfernt. Geeignete Lösungsmittel sind beispielsweise Aromaten wie Benzol oder Toluol, die oben genannten acyclischen Ether, Ester wie Ethylacetat oder chlorhaltige Aliphaten wie Dichlor- oder Trichlormethan.

Das aus der erfindungsgemäßen Umsetzung des exo-Propanons II-exo mit Diphenylmagnesium bzw. mit einem Phenylmagnesiumalkoxid erhaltene Rohprodukt besteht im Wesentlichen aus den zwei diastereomeren exo-Enantiomerenpaaren Ia und Ib des 1-(Bicyclo[2.2.1]-hept-5-en-2-yl)-1-phenyl-3-piperidino-1-propanols (I), wobei das Enantiomerenpaar Ia (Biperiden) bei weitem die Hauptmenge bildet. Das gaschromatographisch bestimmte Verhältnis von Biperiden (Ia) zum Enantiomerenpaar Ib beläuft sich häufig auf etwa 4:1.

Zur Isolierung des Biperidens (Ia) aus dem Diastereomerengemisch wird dieses unter Erwärmen, vorzugsweise bei einer Temperatur von 40 bis 80 °C, insbesondere von 50 bis 70 °C, in einem Gemisch aus Wasser und einem polaren, mit Wasser mischbaren organischen Lösungsmittel gelöst. Geeignete Lösungsmittel sind C₁-C₃-Alkanole, d. h. Methanol, Ethanol, n-Propanol und Isopropanol. Vorzugsweise verwendet man wässriges Isopropanol, besonders bevorzugt 70 bis 95%iges Isopropanol und insbesondere 90%iges Isopropanol. Die hier und im Folgenden gemachten %-Angaben bezüglich des Isopropanolgehalts beziehen sich auf das Volumen des Isopropanols bezogen auf das Gesamtvolumen des wasserhaltigen Lösungsmittels. Zu dieser Lösung wird HCl, beispielsweise in Form einer Lösung von Chlorwasserstoff in einem organischen Lösungsmittel, vorzugsweise in einem der genannten C₁-C₃-Alkanole, bevorzugt in Isopropanol, oder in Form von Salzsäure, zugegeben. HCl wird wenigstens äquimolar bezogen auf das Gemisch der diastereomeren Aminoalkohole, vorzugsweise in einem Überschuss von 5 bis 50 Mol-% und besonders bevorzugt von 5 bis 20 Mol-%, eingesetzt. Die Zugabe erfolgt vorzugsweise bei erhöhter Temperatur, z. B. bei 40 bis 80 °C und insbesondere bei 50 bis 70 °C. Zur Vervollständigung der Reaktion wird nach vollendeter Zugabe das Reaktionsgemisch bei einer Temperatur von 50 °C bis zur Siedetemperatur des Reaktionsgemischs 0,5 bis 3 Stunden belassen, wobei man vorzugsweise rührt. In einer bevorzugten Ausführungsform wird das Reaktionsgemisch zuerst zwei Drittel der Zeit bei 55 bis 65 °C und anschließend ein Drittel der Zeit bei Rückflusstemperatur gerührt. Das Reaktionsgemisch wird anschließend auf eine Temperatur im Bereich von 0 bis 30 °C abgekühlt, gegebenenfalls noch bis zu mehreren Stunden, z. B. bis zu 10 Stunden, vorzugsweise bis zu 5 Stunden, in diesem Temperaturbereich gerührt und dann das gebildete Hydrochlorid in üblicher Weise von der Lösung abgetrennt.

Zur weiteren Aufreinigung des Hydrochlorids wird dieses im Allgemeinen feucht oder trocken in Wasser und einer ausreichenden Menge eines oder mehrerer polarer, mit Wasser begrenzt oder nicht mischbarer Dialkylether mit 4 bis 8 C-Atomen wie Diethylether, tert-Butylmethylether und insbesondere Diisopropylether aufgenommen und das Gemisch mit einer geeigneten Base versetzt. Ausreichende Mengen an organischen Lösungsmitteln sind z. B. 4 bis 10 ml Lösungsmittel pro Gramm trockenen Hydrochlorids. Wasser und organisches Lösungsmittel werden vorzugsweise in einem Volumen-verhältnis im Bereich von 1:2 bis 1:5 eingesetzt.

Geeignete Basen sind Alkali- und Erdalkalihydroxide sowie Alkalicarbonate; besonders bevorzugt werden Natrium- oder Kaliumhydroxid bzw. ihre wässrigen Lösungen und insbesondere Natriumhydroxid bzw. Natronlauge verwendet. Möglich ist aber auch die Verwendung wasserlöslicher organischer Basen, beispielsweise aliphatisch substituierte Amine mit 2 bis 8 C-Atomen. Die Base wird wenigstens äquimolar, vorzugsweise im Überschuss, insbesondere in einem Überschuss von 5 bis 15 Mol-%, bezogen auf das Hydrochlorid, eingesetzt.

Vorzugsweise erfolgt die Umsetzung mit der Base in der Wärme. Hierzu wird vor, während oder bevorzugt nach Zugabe der Base die Mischung auf eine Temperatur im Bereich oberhalb 25 °C bis zur Siedetemperatur des Reaktionsgemischs, vorzugsweise im Bereich von 30 bis 70 °C, bei Verwendung von Diisopropylether als Dialkylether vorzugsweise im Bereich von 40 bis 65 °C, insbesondere von 55 bis 60 °C, erwärmt. Hierbei entstehen in der Regel zwei klare Phasen, die warm, im Falle der Verwendung von Diisopropylether als Dialkylether im oben erwähnten Temperaturbereich, getrennt werden. Die organische Phase wird warm, im Falle der Verwendung von Diisopropylether als Dialkylether im oben erwähnten Temperaturbereich, mit Wasser gewaschen und dann vorzugsweise bei Normaldruck durch Entfernen des Lösungsmittels bis zu einem Gewicht/Volumen-Verhältnis des Produkts zum Lösungsmittel im Bereich von 1:2 bis 1:6, vorzugsweise von 1:3 bis 1:4,5, aufkonzentriert. Beim Abkühlen des Gemischs auf Raumtemperatur oder darunter, vorzugsweise jedoch nicht unter -10 °C, kristallisiert sauberes Biperiden (Ia) aus, das nach üblichen Methoden der Feststoffisolierung, z. B. Abfiltrieren des Feststoffs oder Dekantieren der Mutterlauge, gewonnen wird.

Durch die erfindungsgemäße Verwendung eines Propanons II mit einem exo-Anteil von wenigstens 96 % konnte die Ausbeute an Biperiden (Ia) erheblich gesteigert werden, insbesondere in Kombination mit der oben beschriebenen Aufarbeitung.

Biperiden (Ia) kann anschließend mit einer pharmakologisch verträglichen Säure in üblicher Weise in sein Säureadditionssalz überführt werden. Geeignete Säuren sind beispielsweise Halogenwasserstoffsäuren, insbesondere Chlorwasserstoff bzw. Salzsäure, sowie organische Mono- oder Dicarbonsäuren wie Essigsäure, Oxalsäure, Maleinsäure, Fumarsäure, Milchsäure, Weinsäure, Adipinsäure oder Benzoesäure, weiterhin Phosphorsäure und Schwefelsäure sowie die in "Fortschritte der Arzneimittelforschung, Band 10, S. 224ff, Birkhäuser Verlag, Basel, Stuttgart, 1966" genannten Säuren. Üblicherweise kommt Biperiden (Ia) als Hydrochlorid in den Handel.

Das zur Herstellung des exo-1-(Bicyclo[2.2.1]hept-5-en-2-yl)-3-piperidino-1-propanons II-exo verwendete exo-1-(Bicyclo[2.2.1]-hept-5-en-2-yl)ethanon (III-exo) erhält man durch die Umsetzung von Cyclopentadien und Methylvinylketon in einer Cycloaddition nach Diels und Alder. Eine besonders bevorzugte Methode zur Herstellung von III, bei der man ein Produkt mit hohem Anteil III-exo erhält, wird in der deutschen Patentanmeldung 10124450.9 beschrieben auf deren Offenbarung in vollem Umfang Bezug genommen wird. Die Cycloaddition von Cyclopentadien und Methylvinylketon kann in einem für solche Reaktionen gängigen Lösungsmittel wie Diethylether, Benzol, Toluol oder Xylol oder auch ohne Lösungsmittel durchgeführt werden. Vorzugsweise wird kein Lösungsmittel verwendet. Cyclopentadien und Methylvinylketon werden in der Regel in einem Molverhältnis im Bereich von 3,0:1 bis 0,5:1 eingesetzt. Vorzugsweise werden sie äquimolar oder mit Cyclopentadien im Überschuss umgesetzt, wobei der Überschuss vorzugsweise 50 bis 150 Mol-% beträgt.

Die Reaktion wird in der Regel bei einer Temperatur im Bereich von 0 bis 60 °C, vorzugsweise im Bereich von 10 bis 40 °C durchgeführt.

Niedrig siedende Bestandteile, meist unumgesetzte Edukte, werden in der Regel im Anschluss an die Cycloaddition bei erniedrigtem Druck, vorzugsweise bei 1 bis 150 mbar, destillativ entfernt. Das zurückbleibende Gemisch, das zu etwa 20 % aus exo- und zu etwa 80 % aus endo-1-(Bicyclo[2.2.1]hept-5-en-2-yl)ethanon besteht, wird mit einem Alkali-C₁-C₄-alkoholat umgesetzt. Die Menge an Alkalialkoholat beträgt in der Regel 0,1 bis 5 Gew.-%, vorzugsweise 0,2 bis 2 Gew.-%, bezogen auf das Gesamtgewicht der Mischung. Vorzugsweise wird Natriummethanolat verwendet. Die zur Isomerisierung des Ethanons III erforderliche Temperatur liegt in der Regel im Bereich von 50 bis 110 °C, vorzugsweise im Bereich von 60 bis 100 °C. Hierzu erhitzt man häufig die Mischung unter vermindertem Druck zum Rückfluss, vorzugsweise bei einem Druck von 1 bis 100 mbar und insbesondere bei einem Druck von 5 bis 50 mbar. Man wendet diese Bedingungen in der Regel 10 Minuten bis 5 Stunden, insbesondere 20 Minuten bis 3 Stunden und speziell 0,5 Stunden bis 2 Stunden an und beginnt dann das erhaltene Gemisch fraktioniert zu destillieren, wobei bevorzugt das exo-Isomer von III abdestilliert. Man geht davon aus, dass durch das Entfernen des exo-Isomeren aus dem Gleichgewicht die Isomerisierung des endo-Ethanons zur exo-Form gefördert wird. Die fraktionierte Destillation erfolgt in der Regel über eine Kolonne unter vermindertem Druck, vorzugsweise im Bereich von 1 bis 100 mbar, insbesondere von 1 bis 50 und speziell von 1 bis 20 mbar. Die Destillationstemperatur (Kopftemperatur) wird vorzugsweise auf 50 bis 100 °C und speziell auf 50 bis 80 °C eingestellt. Auf diese Weise erhält man exo-1-(Bicyclo[2.2.1]hept-5-en-2-yl)ethanon (III-exo) in einem Reinheitsgrad, der wenigstens 96 % beträgt.

Durch Redestillation des Destillats erzielt man Reinheitsgrade von bis zu 100 %. Im erfindungsgemäßen Verfahren wird exo-Ethanon III-exo mit einem Reinheitsgrad von wenigstens 96 % eingesetzt.

Die folgenden Beispiele dienen der Veranschaulichung der Erfindung, sind jedoch nicht einschränkend zu verstehen.

### Beispiel

### 1. Herstellung des Ausgangsmaterials

### 1.1 exo-1-(Bicyclo[2.2.1]hept-5-en-2-yl)ethanon (III-exo)

Zu 210,3 g Methylvinylketon wurden zügig 198,3 g Cyclopentadien gegeben. Nach beendeter Zugabe wurde die Reaktionslösung noch eine Stunde bei Raumtemperatur nachgerührt und dann bei einer Temperatur von 58 °C und einem Druck von 20 mbar nicht umgesetztes Edukt destillativ entfernt. Der Eindampfrückstand, hauptsächlich bestehend aus einem Gemisch aus der exo- und der endo-Form von 1-(Bicyclo[2.2.1]hept-5-en-2-yl)ethanon (III) im Verhältnis von 1:4, wurde mit 5 g Natriummethanolat versetzt und eine Stunde bei einem Druck von 10 bis 20 mbar zum Rückfluss erhitzt. Das Reaktionsgemisch wurde anschließend über eine Kolonne bei einer Temperatur von 75 °C und einem Druck von 20 mbar destilliert. Erhalten wurden dabei 298,3 g (73 % der Theorie) exo-1-(Bicyclo[2.2.1-]hept-5-en-2-yl)ethanon (III-exo) in Form eines leicht gelblichen Öls.

### 1.2 exo-1-(Bicyclo[2.2.1]hept-5-en-2-yl)-3-piperidino-1-propanon (II-exo)

### 1.2.1 Herstellung des Trimethylsilylenolethers des exo-Ethanons III-exo exo-({1-[Bicyclo[2.2.1]hept-5-en-2-yl]vinyl}oxy)(trimethyl)silan (IV mit R = Methyl)

68,1 g exo-1-(Bicyclo[2.2.1]hept-5-en-2-yl)ethanon (III-exo), erhalten nach Beispiel 1.1, wurden bei -78 °C innerhalb von 30 Minuten zu 250 ml einer 2M Lithiumdiisopropylamid-Lösung in Tetrahydrofuran/Ethylbenzol/n-Heptan getropft. Das Gemisch wurde eine weitere Stunde bei -78 °C gerührt. Anschließend tropfte man bei -78 °C 67,9 g Trimethylsilylchlorid zügig zu. Das Kühlbad wurde entfernt und die Lösung innerhalb von etwa 1,5 Stunden auf Raumtemperatur aufgetaut. Der gebildete Niederschlag wurde abgenutscht und mit 100 ml n-Hexan gewaschen. Die vereinigten Filtrate wurden mit 250 ml kalter, gesättigter, wässriger Natriumhydrogencarbonatlösung extrahiert und die Phasen getrennt. Danach wurde die wässrige Phase drei Mal mit je 100 ml n-Hexan extrahiert. Die drei organischen Extrakte wurden zu den vereinigten Filtraten gegeben und die so erhaltene organische Phase über Natriumsulfat getrocknet. Das Lösungsmittel wurde entfernt und das erhaltene Rohprodukt durch Destillation bei 95 °C/20 mbar gereinigt. Als Destillat wurden 83,4 g exo-({1-[Bicyclo[2.2.1]-hept-5-en-2-yl]vinyl}oxy)(trimethyl)silan (IV, R = Methyl) in Form eines farblosen Öls erhalten; das sind 80 % der Theorie.

### 1.2.2 Herstellung von exo-1-(Bicyclo[2.2.1]hept-5-en-2-yl)-3-piperidino-1-propanon (II-exo)

83,4 g exo-({1-[Bicyclo[2.2.1]hept-5-en-2-yl]vinyl}oxy)(trimethyl)silan (IV, R = Methyl), erhalten nach Beispiel 1.2.1, wurden in 50 ml N,N-Dimethylformamid bei -25 °C vorgelegt. Im Anschluss wurden 73,5 g N-Methylenpiperidiniumchlorid zugegeben und das Kühlbad wurde entfernt. Das Reaktionsgemisch wurde 30 Minuten gerührt und anschließend zu 250 ml kaltem Wasser gegeben. Der pH-Wert wurde mit verdünnter Salzsäure auf etwa 3 eingestellt und die Lösung drei Mal mit je 75 ml Diisopropylether extrahiert. Die organischen Extrakte wurden verworfen. Die wässrige Phase wurde anschließend mit halbkonzentrierter Natronlauge auf einen pH-Wert von 10 eingestellt und drei Mal mit je 75 ml Diisopropylether extrahiert. Die vereinigten organischen Extrakte wurden über Natriumsulfat getrocknet und das Lösungsmittel wurde im Vakuum entfernt. Erhalten wurden dabei 91,5 g exo-1-(Bicyclo[2.2.1]hept-5-en-2-yl)-3-piperidino-1-propanon (II-exo) in Form eines farblosen Öls; das sind 98 % der Theorie.

### 2. Herstellung von Biperiden (Ia)

103,1 g Dioxan wurden tropfenweise bei 0 °C zu 640 g einer 25%igen Lösung von Phenylmagnesiumchlorid in Tetrahydrofuran gegeben, wobei sich ein weißer Niederschlag bildete. Man rührte 30 Minuten unter Eisbadkühlung und fügte dann zu dem Gemisch 71,5 g 2-Phenylethanol hinzu. Nach weiterem 30-minütigem Rühren unter Eisbadkühlung fügte man unter Eisbadkühlung 91,5 g exo-1-(Bicyclo[2.2.1]hept-5-en-2-yl)-3-piperidino-1-propanon (II-exo), erhalten nach Beispiel 1.2.2, hinzu. Nach beendeter Zugabe wurde das Eisbad entfernt und das Reaktionsgemisch eine weitere Stunde ohne Kühlung gerührt. Das Gemisch wurde anschließend langsam zu 750 ml eiskaltem Wasser gegeben. Danach wurde drei Mal mit je 100 ml Toluol extrahiert. Die vereinigten Extrakte wurden über Natriumsulfat getrocknet und am Rotationsverdampfer eingedampft. Der Eindampfrückstand - 155,5 g eines Gemischs, das im Wesentlichen aus den Formen Ia und Ib von 1-(Bicyclo[2.2.1]hept-5-en-2-yl)-1-phenyl-3-piperidino-1-propanol (I) im Verhältnis (GC) von 4:1 bestand, - wurde in 750 ml Isopropanol bei Rückflusstemperatur gelöst, die Lösung mit 200 ml Wasser versetzt und auf 60 °C abgekühlt. Bei dieser Temperatur wurden 78 ml 5M Salzsäure hinzugefügt. Nach der Säurezugabe wurde eine Stunde bei 60 °C und anschließend eine halbe Stunde bei Rückflusstemperatur nachgerührt. Nach dem Abkühlen auf Raumtemperatur wurden die ausgefallenen Kristalle abgetrennt, mit 150 ml Isopropanol gewaschen und im Vakuum bei 70 °C getrocknet. Das so erhaltene Hydrochlorid (66,5 g) wurde in 375 ml Diisopropylether und 100 ml Wasser unter Rühren mit 78 ml 5M Natronlauge versetzt. Der Ansatz wurde 15 Minuten bei Rückflusstemperatur gerührt, die Wasserphase warm abgetrennt und aus der organischen Phase bei Normaldruck 150 ml Lösungsmittel durch Destillation entfernt. Man ließ den Destillationsrückstand unter Rühren auf Raumtemperatur abkühlen. Nach weiterem einstündigen Abkühlen im Eisbad wurden die ausgefallenen Kristalle abgetrennt, mit 20 ml Diisopropylether gewaschen und im Vakuum bei 40 °C getrocknet. Gewonnen wurden 53,5 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 44 % der Theorie.

### 3. Herstellung des Biperidenhydrochlorids

93,4 g Biperiden (Ia) wurden in 1000 ml Isopropanol durch Erwärmen auf Rückflusstemperatur gelöst. Die Lösung wurde heiß filtriert und der Filter mit 100 ml Isopropanol nachgewaschen. Die vereinigten Filtrate wurden bei 75 °C mit 65 ml 5M Salzsäure versetzt. Anschließend wurde das Gemisch noch 15 Minuten zum Rückfluss erhitzt. Nach dem Abkühlen auf Raumtemperatur wurde noch eine Stunde bei dieser Temperatur gerührt und die ausgefallene Festsubstanz abgesaugt, zwei Mal mit jeweils 50 ml Isopropanol gewaschen und im Vakuum bei 70 °C getrocknet. Erhalten wurden 103,2 g Biperidenhydrochlorid in Form farbloser Kristalle vom Schmelzpunkt 278 bis 280 °C (Ullmanns Enzyklopädie der techn. Chemie, 4. Aufl., Band 21, Verlag Chemie, 1982, S. 627: 278 - 280 °C); das sind 98,9 % der Theorie.

## Patentansprüche

1. Verfahren zur Herstellung von Biperiden (Ia) 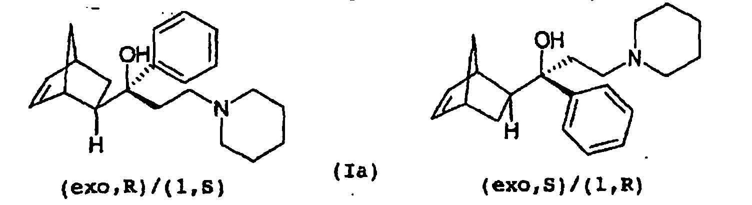 durch Umsetzung von exo-1-(Bicyclo[2.2.1]hept-5-en-2-yl)-3-piperidino-1-propanon (II-exo) 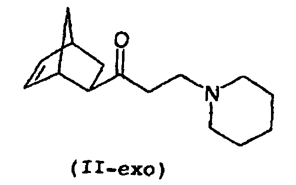 mit einer Phenylmagnesiumverbindung, **dadurch gekennzeichnet, dass** die Herstellung des exo-1-(Bicyclo-[2.2.1]hept-5-en-2-yl)-3-piperidino-1-propanons (II-exo) die folgenden Schritte umfasst:
a) Überführung von exo-1-(Bicyclo[2.2.1]hept-5-en-2-yl)ethanon (III-exo) 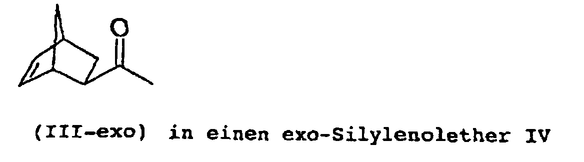 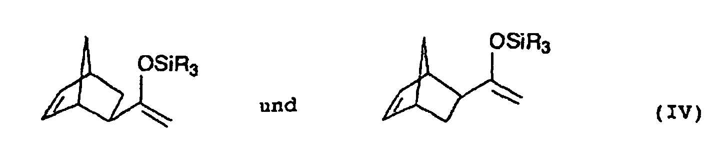 worin R gleich oder verschieden ist und für Alkyl oder Cycloalkyl steht, und
b) Umsetzung des exo-Silylenolethers IV mit einer N-Methylenpiperidinium-Verbindung,
wobei sich die gezeigten Strukturformeln (II-exo) und (III-exo) jeweils auf das Enantiomerenpaar beziehen.

2. Verfahren nach Anspruch 1, **dadurch gekennzeichnet, dass** man in Schritt a) das exo-Ethanon III-exo mit einem Metallamid als Base in das exo-Metallenolat V 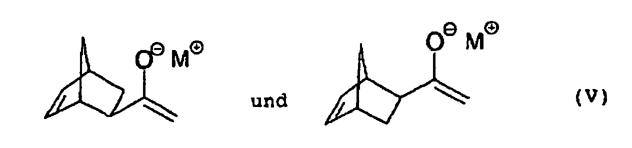 überführt und dieses anschließend mit einer siliziumorganischen Verbindung der Formel R₃Si-X, worin R die zuvor genannte Bedeutung aufweist und X für ein Halogenatom steht, in den exo-Silylenolether IV überführt.

3. Verfahren nach Anspruch 2, **dadurch gekennzeichnet, dass** man als Metallamid ein Alkalimetallamid verwendet.

4. Verfahren nach Anspruch 3, **dadurch gekennzeichnet, dass** man als Alkalimetallamid ein Lithiumamid verwendet.

5. Verfahren nach Anspruch 4, **dadurch gekennzeichnet, dass** man als Lithiumamid Lithiumdiisopropylamid verwendet.

6. Verfahren nach einem der Ansprüche 2 bis 5, **dadurch gekennzeichnet, dass** man das exo-Ethanon III-exo und das Metallamid in einem Molverhältnis von III-exo zu Metallamid im Bereich von 1:1 bis 1:1,5 einsetzt.

7. Verfahren nach einem der vorhergehenden Ansprüche, **dadurch gekennzeichnet, dass** man exo-1-(Bicyclo[2.2.1]hept-5-en-2-yl)ethanon (III-exo) in den exo-Trimethylsilylenolether IV mit R = Methyl überführt.

8. Verfahren nach Anspruch 7, **dadurch gekennzeichnet, dass** man in Schritt a) Trimethylsilylchlorid verwendet.

9. Verfahren nach einem der vorhergehenden Ansprüche, **dadurch gekennzeichnet, dass** man Schritt a) bei einer Temperatur im Bereich von -100 bis 0 °C durchführt.

10. Verfahren nach einem der vorhergehenden Ansprüche, **dadurch gekennzeichnet, dass** man in Schritt b) N-Methylenpiperidiniumchlorid verwendet.

11. Verfahren nach einem der vorhergehenden Ansprüche, **dadurch gekennzeichnet, dass** man in Schritt b) die N-Methylenpiperidinium-Verbindung mit dem exo-Silylenolether IV in einem Molverhältnis im Bereich von 1:1 bis 2:1 umsetzt.

12. Verfahren nach einem der vorhergehenden Ansprüche, **dadurch gekennzeichnet, dass** man Schritt b) bei einer Temperatur im Bereich von -60 bis 10 °C durchführt.

13. Verfahren nach einem der vorhergehenden Ansprüche, **dadurch gekennzeichnet, dass** man das in Schritt b) entstandene Reaktionsgemisch in eine wässrige Lösung überführt und diese bei einem pH-Wert von maximal 6 mit einem mit Wasser begrenzt oder nicht mischbaren Lösungsmittel wäscht, das erhaltene Raffinat bei einem pH-Wert von wenigstens 7,5 mit einem mit Wasser begrenzt oder nicht mischbaren Lösungsmittel extrahiert und das Lösungsmittel aus dem Extrakt entfernt, wobei man exo-1-(Bicyclo[2.2.1]hept-5-en-2-yl)-3-piperidino-1-propanon (II-exo) erhält.

14. Verfahren nach einem der vorhergehenden Ansprüche, **dadurch gekennzeichnet, dass** man als Phenylmagnesiumverbindung Diphenylmagnesium oder eine Phenylmagnesiumverbindung der allgemeinen Formel 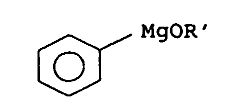 verwendet, wobei R' für C₁-C₄-Alkyl, C₄-C₆-Cycloalkyl, C₄-C₆-Cycloalkyl-C₁-C₄-alkyl, Phenyl-C₁-C₄-alkyl, substituiertes Phenyl-C₁-C₄-alkyl, Heteroaryl, Heteroaryl-C₁-C₄-alkyl oder Benzhydryl steht.

15. Verfahren nach einem der vorhergehenden Ansprüche, **dadurch gekennzeichnet, dass** man zur Gewinnung von Biperiden (Ia) aus der Umsetzung des exo-1-(Bicyclo[2.2.1]hept-5-en-2-yl)-3-piperidino-1-propanons (II-exo) mit der Phenylmagnesiumverbindung das hierbei gebildete Gemisch diastereomerer 1-(Bicyclo[2.2.1]hept-5-en-2-yl)-1-phenyl-3-piperidino-1-propanole
- in wässrigem Isopropanol in das Hydrochlorid überführt und dieses isoliert,
- dieses in Wasser und Diisopropylether mit einer Base umsetzt,
- die wasserhaltige Phase in der Wärme abtrennt,
- aus der organischen Phase einen Teil des Diisopropylethers entfernt und nach dem Abkühlen durch Abtrennen des Feststoffs von der Mutterlauge Biperiden (Ia) isoliert.

16. exo-({1-[Bicyclo[2.2.1]hept-5-en-2-yl]vinyl}oxy)silane der Formel IV 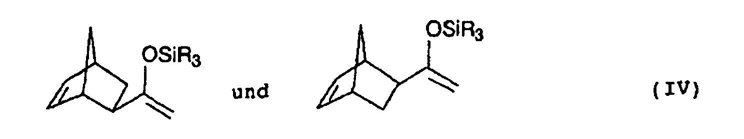 worin R gleich oder verschieden ist und für Alkyl oder Cycloalkyl steht.

## Claims

1. Method for producing biperidene (Ia) 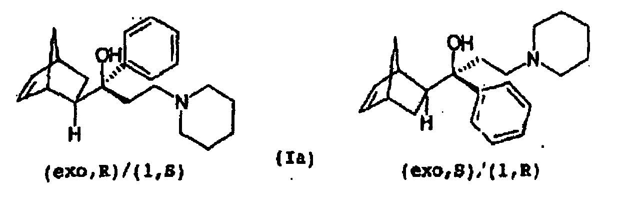 by reaction of exo-1-(bicyclo[2.2.1]hept-5-en-2-yl)-3-piperidino-1-propanone (II-exo) 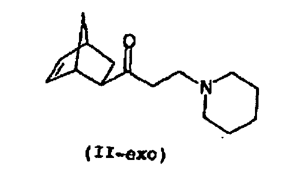 with a phenylmagnesium compound, **characterized in that** the producdon of the exo-1-(bicyclo[2.2.1]hept-5-en-2-yl)-3-piperidino-1-propanone (II-exo) comprises the following steps:
a) conversion of exo-1-[bicyclo[2.2.1]hept-5-en-2-yl)ethanone (III-exo) 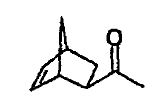 into an exo-silyl enol ether IV 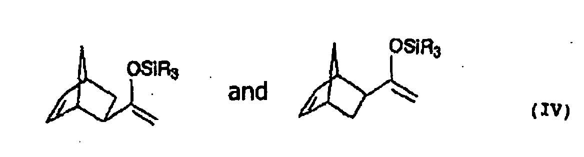 wherein R is the same or different and is alkyl or cycloalkyl, and
b) reaction of the exo-silyl enol ether IV with a N-methylpiperidinium compound, wherein the shown structural formulae (II-exo) and (III-exo) each relate to the pair of enantiomers.

2. Method according to claim 1, **characterized in that** in step a), the exo-ethanone III-exo is converted with a metal amide as a base into the exo-metal enolate V 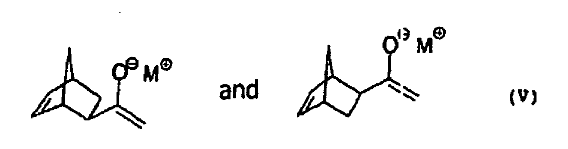 which is then converted into the exo-silyl enol ether IV with a silicon organic compound of formula R₃Si-X, wherein R has the above meaning and X is a halogen atom.

3. Method according to claim 2, **characterized** n that an alkali metal amide is used as metal amide.

4. Method according to claim 3, **characterized** n that a lithium amide is used as a alkali metal amide.

5. Method according to claim 4, **characterized** n that lithium diisopropyl amide is used as lithium amide.

6. Method according to any of claims 2 to 5, **characterized in that** the exo-ethanone III-exo and the metal amide are used in a mole ratio of III exo to metal amide In the range of 1 : 1 to 1 : 1.5.

7. Method according to any of the preceding claims, **characterized In that** the exo-1-(bicyclo[2.2.1]hept-5-en-2-yl)ethanone (III-exo) is converted into the exo-trimethylsilyl enol ether IV with R = methyl.

8. Method according to claim 7, **characterized in that** trimethylsilyl chloride is used in step a).

9. Method according to any of the preceding claims, **characterized in that** step a) is carried out at a temperature In the range of -100 to 0°C.

10. Method according to any of the preceding claims, **characterized in that** N-methyleneplperidinium chloride is used in step b).

11. Method according to any of the preceding claims, **characterized in that** in step b), the N-methylenepiperidinium compound is reacted with the exo-silyl enol ether IV in a mole ratio in the range from 1 : 1 to 2 : 1.

12. Method according to any of the preceding claims, **characterized in that** step b) is carried out at a temperature in the range from -50 to 10°C.

13. Method according to any of the preceding claims, **characterized in that** the reaction mixture resulting from step b) is converted into an aqueous solution and said solution is washed at a pH of at maximum 6 with a solvent which is miscible with water to only a limited extent or immiscible with water, the obtained raffinate is extracted at a pH of at least 7.5 with a solvent that is miscible with water to only a limited extent or is immiscible with water and the solvent is removed from the extract, whereby the exo-1-(bicyclo[2.2.1]hept-5-en-2-yl)-3-piperidino-1-propanone (II-exo) is obtained.

14. Method according to any of the preceding claims, **characterized in that** diphenylmagnesium or a phenylmagnesium compound of the general formula 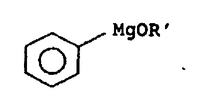 is used as phenylmagnesium compound, wherein R^{'} is C₁-C₄ alkyl, C₄-C₆ cycloalkyl, C₄-C₆ cycloalkyl-C₁-C₄ alkyl, phenyl C₁-C₄ alkyl, substituted phenyl C₁-C₄ alkyl, heteroaryl, heteroaryl C₁-C₄ alkyl or benzhydryl.

15. Method according to any of the preceding claims, **characterized in that** for obtaining biperiden (Ia) from the reaction of exo-1-(bicyclo[2.2.1]hept-5-en-2-yl)-3-piperidino-1-propanone (II-exo) with the phenylmagnesium compound, the thereby formed mixture of diastereomeric 1-(bicyclo[2.2.1]hept-5-en-2-yl)-1-phenyl-3-plperidino-1-propanoles
- is converted into the hydrochloride in aqueous isopropanole and isolated,
- which is reacted with a base in water and diisopropyl ether,
- the aqueous phase is separated in heat,
- a part of the diisopropyl ether is removed from the organic phase and after cooling, biperidene (Ia) is isolated by separating the solid from the mother liquor.

16. Exo-({1-(bicyclo[2.2.1]hept-5-en-2-yl]vinyl}oxy) silane of the formula IV 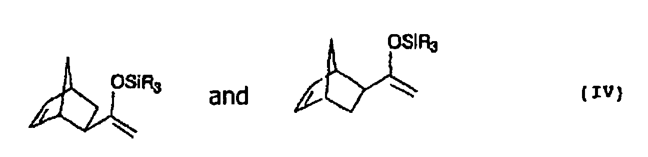 wherein R is the same or different and is alkyl or cycloalkyl.

## Revendications

1. Procédé de préparation du bipéridène (Ia) 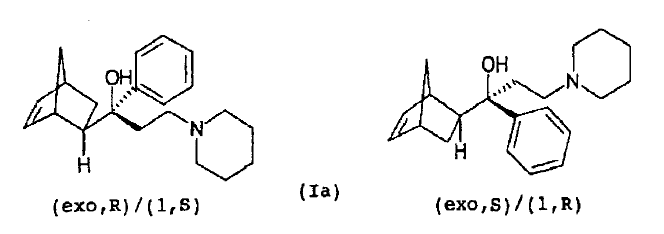 par réaction d'exo-1-(Bicyclo [2.2.1] hept-5-èn-2-yl)-3-pipéridino-1-propanone (II-exo) 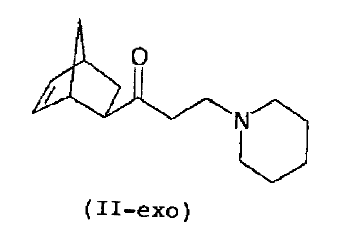 avec un composé phénylmagnésien, **caractérisé en ce que** la préparation de l'exo-1-(Bicyclo [2.2.1] hept-5-èn-2-yl)-3-pipéridino-1-propanone (II-exo) comprend les étapes suivantes :
a) conversion de l'exo-1-(Bicyclo [2.2.1] hept-5-èn-2-yl) éthanone (III-exo) 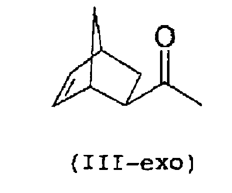 en un exo-silylénoléther IV 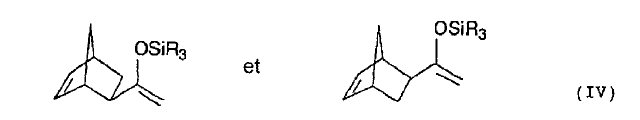 dans lequel les R sont identiques ou différents et représentent un alkyle ou un cycloalkyle, et
b) réaction de l'exo-silylénoléther IV avec un composé de N-méthylène pipéridinium,
dans lequel les formules développées (II-exo) et (III-exo) présentées se rapportent respectivement à la paire d'énantiomères.

2. Procédé selon la revendication 1, **caractérisé en ce que** dans l'étape a) on convertit l'exo-éthanone III-exo avec un amide métallique en tant que base, en l'exo-énolate métallique V 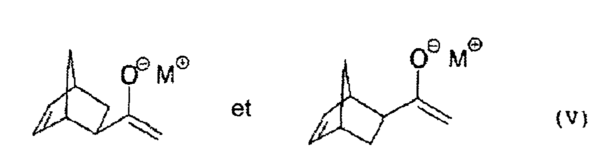 et **en ce que** l'on convertit par la suite celui-ci avec un composé organique du silicium de formule R₃Si-X, dans laquelle R présente la signification mentionnée auparavant et X représente un atome d'halogène, en l'exo-silylénoléther IV.

3. Procédé selon la revendication 2, **caractérisé en ce que** l'on utilise comme amide métallique un amide de métal alcalin.

4. Procédé selon la revendication 3, **caractérisé en ce que** l'on utilise comme amide de métal alcalin un amide de lithium.

5. Procédé selon la revendication 4, **caractérisé en ce que** l'on utilise comme amide de lithium du diisopropylamide de lithium.

6. Procédé selon l'une des revendications 2 à 5, **caractérisé en ce que** l'on utilise l'exo-éthanone III-exo et l'amide métallique dans un rapport molaire du III-exo sur l'amide métallique dans la gamme de 1 : 1 à 1 : 1,5.

7. Procédé selon l'une des revendications précédentes, **caractérisé en ce que** l'on convertit de l'exo-1-(Bicyclo [2.2.1] hept-5-èn-2-yl) éthanone (III-exo) en l'exo-triméthylsilylénoléther IV avec R = méthyle.

8. Procédé selon la revendication 7, **caractérisé en ce que** dans l'étape a) on utilise du chlorure de triméthylsilyle.

9. Procédé selon l'une des revendications précédentes, **caractérisé en ce que** l'on réalise l'étape a) à une température dans la gamme de -100 à 0 °C.

10. Procédé selon l'une des revendications précédentes, **caractérisé en ce que** l'on utilise dans l'étape b) du chlorure de N-méthylène pipéridinium.

11. Procédé selon l'une des revendications précédentes, **caractérisé en ce que** dans l'étape b) on fait réagir le composé de N-méthylène pipéridinium avec l'exo-silylénoléther IV dans un rapport molaire dans la gamme de 1 : 1 à 2 : 1.

12. Procédé selon l'une des revendications précédentes, **caractérisé en ce que** l'on réalise l'étape b) à une température dans la gamme de -60 à 10 °C.

13. Procédé selon l'une des revendications précédentes, **caractérisé en ce que** l'on convertit le mélange réactionnel résultant de l'étape b) en une solution aqueuse, et **en ce que** l'on lave celle-ci à une valeur de pH au maximum de 6 avec un solvant miscible de façon limitée ou non miscible avec l'eau, **en ce que** l'on extrait le raffinat obtenu à une valeur de pH d'au moins 7,5 avec un solvant miscible de façon limitée ou non miscible avec l'eau, et **en ce que** l'on élimine le solvant de l'extrait, dans lequel on obtient de l'exo-1-(Bicyclo [2.2.1] hept-5-èn-2-yl)-3-pipéridino-1-propanone (II-exo).

14. Procédé selon l'une des revendications précédentes, **caractérisé en ce que** comme composé phénylmagnésien on utilise du diphénylmagnésium ou un composé phénylmagnésien de formule générale 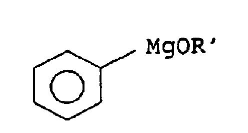 dans lequel R' représente un alkyle en C₁ à C₄, un cycloalkyle en C₄ à C₆, un (cycloalkyle en C₄ à C₆) - (alkyle en C₁ à C₄), un phényl-(alkyle en C₁ à C₄), un phényl-(alkyle en C₁ à C₄) substitué, un hétéroaryle, un hétéroaryl-(alkyle en C₁ à C₄) ou un benzhydryle.

15. Procédé selon l'une des revendications précédentes, **caractérisé en ce que**, pour l'obtention du bipéridène (Ia) à partir de la réaction de l'exo-1-(Bicyclo [2.2.1] hept-5-èn-2-yl)-3-pipéridino-1-propanone (II-exo) avec le composé phénylmagnésien, le mélange ainsi formé des diastéréoisomères du 1-(Bicyclo [2.2.1] hept-5-én-2-yl)-1-phényl-3-pipéridino-1-propanol
- est converti dans de l'isopropanol aqueux en le chlorhydrate et celui-ci est isolé,
- celui-ci est mis à réagir avec une base dans de l'eau et de l'éther diisopropylique,
- la phase contenant de l'eau est séparée par la chaleur,
- une partie de l'éther düsopropylique est éliminée de la phase organique, et du bipéridène (Ia) est isolé après le refroidissement par séparation de la matière solide du percolat mère.

16. Exo-({1-[Bicyclo [2.2.1] hept-5-èn-2-yl] vinyl} oxy) silane de formule IV 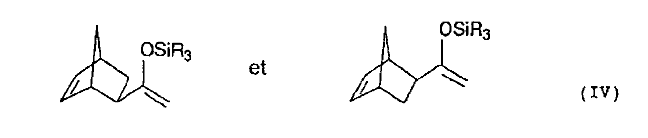 dans lequel les R sont identiques ou différents et représentent un alkyle ou un cycloalkyle.
